# FASCICULE DE BREVET EUROPEEN

(11) **EP 3 801 398 B1**
(45) Date de publication et mention de la délivrance du brevet: **28.06.2023**
(21) Numéro de dépôt: 19737811.0
(22) Date de dépôt: 23.05.2019
(51) Int. Cl.: A61F 2/46, A61B 17/02

(54) **INSTRUMENT CHIRURGICAL POUR UN ÉQUILIBRAGE DU GENOU**
CHIRURGISCHES INSTRUMENT FÜR DAS KNIEAUSGLEICH
SURGICAL INSTRUMENT FOR KNEE BALANCING

(30) Priorité: 24.05.2018 FR 1854341
(43) Date de publication de la demande: 14.04.2021
(73) Titulaire: Amplitude, 26000 Valence (FR)
(72) Inventeur: LERAT, Bernard, 26000 VALENCE (FR); THIBAUT, Sylvain, 26320 SAINT MARCEL LES VALENCES (FR); GOUBET, Nicolas, 38190 BERNIN (FR)
(74) Mandataire: Germain Maureau
(86) Numéro de dépôt international: PCT/FR2019/051184
(87) Numéro de publication internationale: WO 2019/224488

(56) Documents cités:
- EP-A1- 2 277 460
- EP-A2- 0 809 969
- WO-A2-2010/111678
- US-A1- 2004 122 441
- US-A1- 2016 135 825

## Description

La présente invention se rapporte à un instrument chirurgical pour un équilibrage du genou dans le cadre d'une préparation à la pose chirurgicale d'une prothèse totale de genou.

Lors de la pose d'une prothèse totale de genou, le chirurgien doit faire particulièrement attention aux deux points suivants :
- l'alignement de la jambe en extension où le centre hanche, le centre genou et le centre cheville tendent à être totalement ou partiellement alignés après l'opération ;
- la tension ligamentaire au niveau des ligaments médial et latéral afin de garantir une bonne stabilité de l'articulation.

L'invention se rapporte plus particulièrement au second point en proposant un instrument chirurgical permettant d'effectuer un équilibrage ligamentaire en préparation à la pose d'une prothèse totale de genou.

Actuellement, il est connu de gérer ce second point en mettant l'articulation du genou en tension avec des cales ou avec un tenseur mécanique puis en évaluant manuellement la tension ligamentaire, le résultat obtenu étant alors subjectif.

L'état de la technique peut être illustré par les enseignements des documents US9592133 et US2016/0135825 qui divulguent chacun un instrument chirurgical comprenant un plateau tibial et un plateau fémoral pivotant relativement au plateau tibial qui est statique, ainsi qu'un système de réglage procurant une fonction de distraction en permettant un réglage de l'écartement entre le plateau fémoral et le plateau tibial.

Avec ce type d'instrument, il est alors possible de faire pivoter le plateau fémoral relativement au plateau tibial, de sorte que le chirurgien puisse effectuer ensuite des mouvements de varus valgus pour obtenir une estimation de la stabilité de l'articulation. Cependant, il est à noter que le pivotement du plateau fémoral relativement au plateau tibial est libre, ce qui permet certes à l'instrument d'être toujours dans une situation où la tension médiale est égale à la tension latérale, mais avec l'inconvénient de ne pas permettre une immobilisation de l'angle de pivotement entre le plateau fémoral et le plateau tibial à différentes valeurs, hormis un éventuel verrouillage de l'angle de pivotement limité à la seule valeur de zéro degré pour immobiliser un parrallélisme entre le plateau fémoral et le plateau tibial.

L'état de la technique peut également être illustré par l'enseignement du document EP 2 277 460 qui divulgue un instrument chirurgical comprenant un plateau tibial, un plateau fémoral et un élément mobile monté mobile en translation sur une base solidaire du plateau tibial et sur lequel est monté pivotant le plateau fémoral selon un axe antéro-postérieur, ainsi qu'un système de blocage réversible conçu pour immobiliser de manière réversible le pivotement du plateau fémoral par rapport à l'élément mobile afin de fixer l'angle de pivotement du plateau fémoral relativement au plateau tibial à différentes valeurs d'angle de pivotement.

Dans ce document EP 2 277 460, le système de blocage réversible est formé d'un téton muni d'une empreinte femelle pour coopérer avec un tournevis et d'un alésage dans lequel est vissée une vis traversant une gorge arquée pour un blocage par serrage, de sorte que le chirurgien peut fixer la valeur de l'angle de pivotement avec son tournevis, selon différents paramètres dans le cadre d'une mesure peropératoire, c'est-à-dire avant l'opération. Cependant, le téton et l'alésage s'étendent de manière coaxiale selon une direction parallèle à l'axe antéro-postérieur, de sorte que le chirurgien peut certes fixer avant l'opération la valeur de l'angle de pivotement avec son tournevis, mais il lui sera difficile d'ajuster cet angle de pivotement en cours d'opération, en ressentant physiquement pendant l'opération l'angle de pivotement qui sera le plus adapté.

La présente invention a notamment pour but de résoudre en tout ou partie l'inconvénient précité en proposant un instrument chirurgical qui permet à la fois un ajustement libre de l'équilibrage ligamentaire jambe en flexion et un contrôle objectif de l'équilibrage ligamentaire jambe en flexion et également jambe en extension.

Un autre but de l'invention est de permettre un positionnement d'un élément de guidage comprenant plusieurs orifices de guidage pour des perçages dans le fémur, afin de permettre de finaliser les coupes fémorales directement basées sur l'équilibrage contrôlé.

A cet effet, elle propose un instrument chirurgical pour un équilibrage du genou dans le cadre d'une préparation à la pose chirurgicale d'une prothèse totale de genou, ledit instrument chirurgical comprenant :
- un élément tibial statique incluant une base solidaire d'un plateau tibial prévu pour venir en appui sur une extrémité proximale reséquée du tibia ;
- un élément fémoral incluant un plateau fémoral prévu pour venir en appui sur les condyles distaux ou postérieurs du fémur ou sur une extrémité distale reséquée du fémur ou sur une cale de compensation ;
- un élément mobile incluant un guide monté mobile en translation sur la base de l'élément tibial statique selon un axe longitudinal, et dans lequel l'élément fémoral est monté pivotant sur ledit élément mobile selon un axe antéro-postérieur, de sorte que le plateau fémoral est pivotant autour dudit axe antéro-postérieur relativement au plateau tibial qui est statique ;
- un système de réglage accouplé entre le guide de l'élément mobile et la base de l'élément tibial statique pour un réglage de la mobilité dudit guide relativement à ladite base afin de procurer une fonction de distraction en permettant un réglage de l'écartement entre le plateau fémoral et le plateau tibial ;

dans lequel l'élément mobile comprend un bras transversal solidaire du guide, et l'élément fémoral comprend un bras fémoral solidaire du plateau fémoral ;
et dans lequel l'instrument chirurgical comprend en outre un système de blocage réversible accouplé entre le bras transversal de l'élément mobile et le bras fémoral de l'élément fémoral pour immobiliser de manière réversible le pivotement de l'élément fémoral par rapport à l'élément mobile afin de fixer l'angle de pivotement du plateau fémoral relativement au plateau tibial à différentes valeurs d'angle de pivotement ;
ledit instrument chirurgical selon l'invention étant remarquable en ce que le système de blocage réversible comprend :
   - une mâchoire qui est prévue sur une extrémité libre du bras fémoral, et qui comprend deux mors en vis-à-vis mobiles entre une position serrée et une position desserrée ;
   - un rail qui est prévu sur une extrémité libre du bras transversal de l'élément mobile et qui est engagé à l'intérieur de la mâchoire entre les deux mors ;
   - un élément de serrage coopérant avec la mâchoire pour déplacer les mors entre la position serrée et la position desserrée, où dans la position serrée l'élément fémoral est immobilisé dans son pivotement par rapport à l'élément mobile, et où dans la position desserrée l'élément fémoral est autorisé à pivoter par rapport à l'élément mobile ;
en ce que ledit élément de serrage comprend une vis de serrage vissée dans un orifice de serrage qui traverse les deux mors, de sorte que le vissage de la vis de serrage déplace les mors vers la position serrée et le dévissage de la vis de serrage déplace les mors vers la position desserrée ;
et en ce que la vis de serrage et l'orifice de serrage s'étendent selon un axe transversal orthogonal à l'axe antéro-postérieur.

Ainsi, l'invention permet d'objectiver le positionnement angulaire en rotation du condyle prothétique lors de la pose d'une prothèse totale de genou avec une instrumentation conventionnelle. Lors de la mise en tension, le plateau fémoral pivote librement relativement au plateau tibial, ce qui tend à équilibrer les forces. Il est à noter qu'avec un tel instrument chirurgical selon l'invention, il est possible, voire préférentiel, que le plateau fémoral soit libre de pivoter au début de l'opération, à la différence de la démarche décrite dans le document EP 2 277 460 qui préconise de bloquer le pivotement du plateau fémoral à une valeur fixe en préalable à l'opération.

Le chirurgien peut ensuite se satisfaire de l'équilibre obtenu mécaniquement ou il peut ajuster l'équilibrage en faisant pivoter le bras fémoral, et donc le plateau fémoral en se servant d'un tournevis de serrage engagé dans la vis de serrage comme bras de levier pour faire pivoter l'élément fémoral par rapport à l'élément mobile lorsque les mors sont en position desserrée. L'orientation de la vis de serrage selon un axe transversal orthogonal à l'axe antéro-postérieur, permet au chirurgien d'exercer aisément un effort sur le plateau fémoral à la manière d'un bras de levier, pour le faire pivoter, jusqu'à ce qu'il « ressente » le bon angle de pivotement qu'il va pouvoir figer en serrant la vis de serrage.

L'ajustement ainsi obtenu est donc figé au moyen du système de blocage réversible pour verrouiller le pivotement du plateau fémoral, à un angle nul ou à différents autres angles en fonction de l'ajustement ligamentaire recherché par le chirurgien.

Avantageusement, le bras transversal de l'élément mobile se termine par une extrémité libre munie d'un premier élément de repérage, et le bras fémoral de l'élément fémoral se termine par une extrémité libre munie d'un second élément de repérage, où le premier élément de repérage et le second élément de repérage comprennent une échelle de graduations réparties autour de l'axe antéro-postérieur et un repère de référence en correspondance visuelle avec l'échelle de graduations, de manière à mesurer une valeur d'angle de pivotement du plateau fémoral relativement au plateau tibial.

Selon une caractéristique, la mâchoire est formée d'une glissière fendue dans laquelle est prévue une fente entre les deux mors.

L'invention concerne également un système chirurgical comprenant un tel instrument chirurgical et comprenant en outre un tournevis de serrage apte à coopérer avec la vis de serrage pour assurer son vissage/dévissage, et apte aussi à former un bras de levier, une fois enclenché avec la vis de serrage, afin de faire pivoter l'élément fémoral par rapport à l'élément mobile lorsque les mors sont en position desserrée.

Le chirurgien peut donc ajuster l'équilibrage en se servant de l'effet bras de levier offert par le tournevis de serrage enclenché avec la vis de serrage, l'ajustement ainsi obtenu étant ensuite figé en vissant la vis de serrage jusqu'à verrouiller le pivotement du plateau fémoral, à un angle nul ou à différents autres angles en fonction de l'ajustement ligamentaire recherché par le chirurgien.

Selon une possibilité de l'invention, le guide comprend une crémaillère formée d'une série de dents, et le système de réglage comprend un pignon qui est monté pivotant sur la base de l'élément tibial statique et qui est en engrènement avec la crémaillère.

Dans un premier mode de réalisation de l'invention, le système de réglage comprend un mécanisme de verrouillage réversible accouplé entre la base de l'élément tibial statique et le pignon pour verrouiller de manière réversible la rotation du pignon et par conséquent la mobilité du guide relativement à la base, afin de fixer l'écartement entre le plateau fémoral et le plateau tibial.

Conformément à une caractéristique de ce premier mode de réalisation, le mécanisme de verrouillage réversible comprend une gâchette montée mobile sur la base de l'élément tibial statique entre une position de verrouillage dans laquelle la gâchette coopère avec le pignon pour le verrouiller en rotation, et une position de déverrouillage dans laquelle la gâchette ne coopère pas avec le pignon qui est libre de pivoter.

Dans un second mode de réalisation de l'invention, le système de réglage comprend un mécanisme de verrouillage réversible accouplé entre la base de l'élément tibial statique et la crémaillère pour verrouiller directement et de manière réversible la mobilité du guide relativement à la base, afin de fixer l'écartement entre le plateau fémoral et le plateau tibial.

Ainsi, à la différence du ce premier mode de réalisation, le mécanisme de verrouillage réversible du second mode de réalisation agit directement sur la crémaillère, et non pas sur le pignon, ce qui a l'avantage de procurer une stabilité mécanique accrue.

Conformément à une caractéristique de ce second mode de réalisation, le mécanisme de verrouillage réversible comprend au moins un pêne monté pivotant sur la base entre une position de verrouillage dans laquelle le pêne est en engagement entre deux dents de la crémaillère pour la verrouiller en translation, et une position de déverrouillage dans laquelle le pêne ne coopère pas avec les dents de la crémaillère pour l'autoriser à translater.

Avantageusement, le mécanisme de verrouillage réversible de ce second mode de réalisation comprend deux pênes montés pivotant sur la base entre une position de verrouillage et une position de déverrouillage, où lesdits pênes sont décalés l'un de l'autre selon l'axe longitudinal d'un écart correspondant à la moitié d'un pas de la crémaillère, le pas de la crémaillère correspondant à l'écartement entre deux dents successives, de sorte que lorsqu'un pêne est en position de verrouillage entre deux dents de la crémaillère alors l'autre pêne est en position de déverrouillage en étant au contact d'un sommet d'une dent de la crémaillère.

De cette manière, il est possible de bloquer la distraction par incréments d'un demi-pas de la crémaillère (et non pas d'un pas entier), ce qui permet aussi d'avoir une crémaillère avec un pas plus élevé et donc avec une résistance mécanique accrue.

La présente invention concerne également les caractéristiques ci après :
- le guide est muni d'un premier élément de mesure ;
- la base de l'élément tibial statique présente une fenêtre à travers laquelle est visible le premier élément de mesure du guide, et ladite base présente en outre un second élément de mesure placé sur le côté de la fenêtre ;
- le premier élément de mesure et le second élément de mesure comprennent une échelle de graduations réparties le long de l'axe longitudinal et un repère de référence en correspondance visuelle avec l'échelle de graduations, de manière à mesurer une valeur d'écartement entre le plateau fémoral et le plateau tibial.

L'invention se rapporte également à un système chirurgical comprenant un instrument chirurgical selon l'invention et comprenant en outre un capteur de force adapté pour être placé de manière amovible sur une face supérieure du plateau fémoral, opposée à une face inférieure qui fait face au plateau tibial, afin de détecter une ou plusieurs forces exercées par les condyles du fémur sur le plateau fémoral.

Avantageusement, il est prévu un appareil de mesure raccordé au capteur de force, par une voie non filaire ou filaire, et prévu pour recevoir les mesures effectuées par le capteur de force, dans lequel cet appareil de mesure est monté de manière amovible sur la base de l'élément tibial statique.

Accessoirement, cet appareil de mesure est muni d'un afficheur procurant un affichage des mesures effectuées par le capteur de force.

En variante, cet appareil de mesure est raccordé à un afficheur distant, notamment par voie radio, cet afficheur distant procurant un affichage des mesures effectuées par le capteur de force.

L'invention se rapporte aussi à un système chirurgical comprenant un instrument chirurgical selon l'invention et comprenant en outre un élément de guidage monté de manière amovible sur la base de l'élément tibial statique, ledit élément de guidage comprenant plusieurs orifices de guidage pour des perçages dans le fémur ; de tels perçages pouvant servir ensuite pour fixer les guides de coupe fémorale.

D'autres caractéristiques et avantages de la présente invention apparaîtront à la lecture de la description détaillée ci-après, d'un exemple de mise en oeuvre non limitatif, faite en référence aux figures annexées dans lesquelles :
- la figure 1 est une vue schématique en perspective d'un système chirurgical selon un premier mode de réalisation de l'invention et au complet, comprenant un instrument chirurgical selon ce premier mode de réalisation de l'invention, un capteur de force, un appareil de mesure raccordé au capteur de force, une cale de compensation, et un élément de guidage ;
- la figure 2 est une vue schématique en perspective d'un élément fémoral seul pour l'instrument chirurgical visible en figure 1 ;
- les figures 3 et 4 sont des vues schématiques en perspective, selon deux angles de vue distincts, de l'instrument chirurgical visible en figure 1 ;
- la figure 5 est une vue schématique en perspective d'un élément mobile seul pour l'instrument chirurgical visible sur les figures 1, 3 et 4 ;
- la figure 6 est une vue schématique en perspective d'un élément tibial statique seul pour l'instrument chirurgical visible sur les figures 1, 3 et 4 ;
- la figure 7 est une vue schématique en perspective d'une gâchette couplé à un pignon pour l'instrument chirurgical visible sur les figures 1, 3 et 4 ;
- la figure 8 est une vue schématique en perspective de la cale de compensation seule pour le système chirurgical de la figure 1 ;
- la figure 9 est une vue schématique en perspective de l'élément de guidage seul pour le système chirurgical de la figure 1 ;
- la figure 10 est une vue schématique en perspective de l'ensemble capteur de force et appareil de mesure seul pour le système chirurgical de la figure 1 ;
- la figure 11 est une vue schématique en perspective du système chirurgical de la figure 1, sans l'élément de guidage, et avec un tournevis de serrage ;
- la figure 12 est une vue schématique en perspective du système chirurgical de la figure 1, sans l'ensemble capteur de force et appareil de mesure ;
- la figure 13 est une vue schématique en perspective d'un instrument chirurgical selon un seconde mode de réalisation de l'invention, avec certains éléments telle que la base de l'élément tibial en mode transparent ;
- la figure 14 est une vue schématique en perspective de l'instrument chirurgical de la figure 13, sans l'élément tibial ;
- la figure 15 est une vue schématique de côté de l'instrument chirurgical de la figure 13 avec certains éléments telle que la base de l'élément tibial en mode transparent ; et
- la figure 16 est une vue schématique en perspective d'un pêne seul du mécanisme de verrouillage réversible employé dans l'instrument chirurgical de la figure 13.

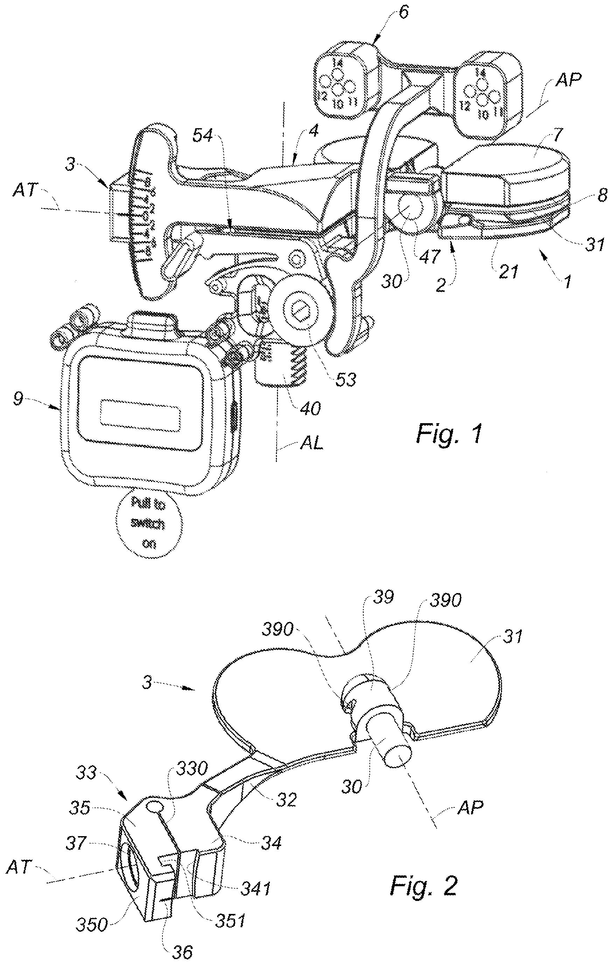

En référence aux figures 1, 3, 4, 11 et 12, un instrument chirurgical 1 selon un premier mode de réalisation de l'invention, comprend principalement les éléments suivants :
- un élément tibial statique 2 incluant une base 20 solidaire d'un plateau tibial 21 prévu pour venir en appui sur une extrémité reséquée du tibia ;
- un élément fémoral 3 incluant un plateau fémoral 31 prévu pour venir en appui sur les condyles distaux ou postérieurs du fémur ou sur une extrémité distale reséquée du fémur ;
- un élément mobile 4 incluant un guide 40 monté mobile en translation sur la base 20 de l'élément tibial statique 2 selon un axe longitudinal AL ;
- un système de réglage 5 accouplé entre le guide 40 de l'élément mobile 4 et la base 20 de l'élément tibial statique 2 pour un réglage de la mobilité du guide 40 relativement à la base 20 afin de procurer une fonction de distraction en permettant un réglage de l'écartement entre le plateau fémoral 31 et le plateau tibial 21, cet écartement étant pris selon l'axe longitudinal AL.

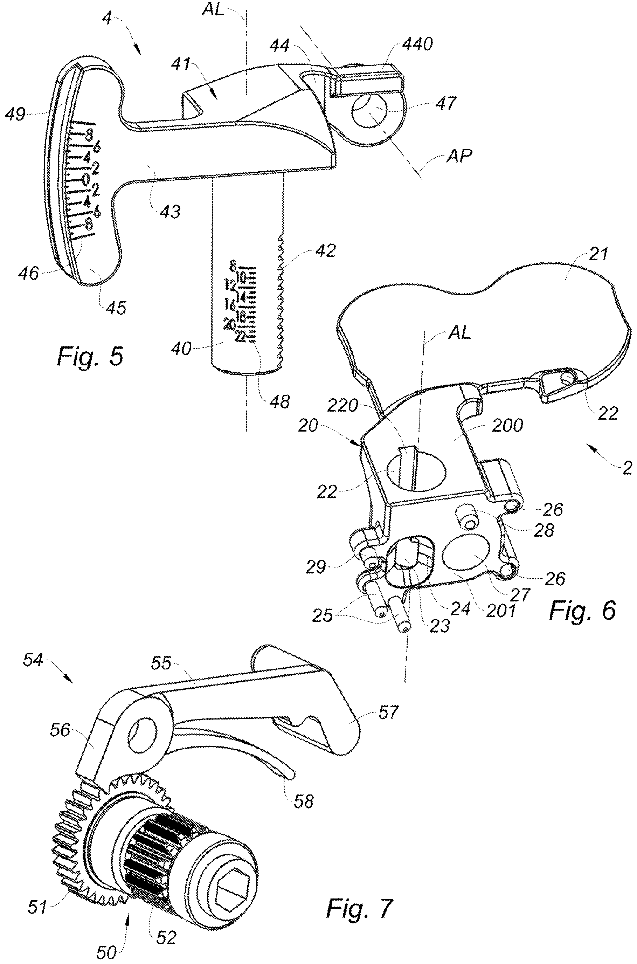

En référence à la figure 6, l'élément tibial statique 2 est formé d'une pièce monobloc et comprend principalement la base 20 et le plateau tibial 21. Cet élément tibial statique 2 est prévu pour être placé de manière statique sur l'extrémité réséquée du tibia et, à ce titre, il peut présenter un orifice de fixation 22 ménagé en bordure du plateau tibial qui permet de stabiliser l'élément tibial statique 2 sur l'extrémité réséquée du tibia avec un clou.

La base 20 présente une face supérieure 200, parallèle au plateau tibial 21, et dans laquelle est ménagée un orifice de guidage 22 s'étendant selon l'axe longitudinal AL et traversant la base 20 sur toute sa hauteur en débouchant sur une face inférieure, opposée à la face supérieure 200. Cet orifice de guidage 22 est prévu pour recevoir intérieurement le guide 40 de l'élément mobile 4, afin de le guider en coulissement. Cet orifice de guidage 22 est muni d'une rainure 220 pour empêcher la rotation du guide 40 à l'intérieur de l'orifice de guidage 22.

La base 20 présente une face frontale 201 dans laquelle est ménagée une fenêtre 23 débouchant dans l'orifice de guidage 22 et à travers laquelle est visible le guide 40 une fois en place à l'intérieur de l'orifice de guidage 22. La base 20 présente en outre un repère 24 placé sur le côté de la fenêtre 23 ; ce repère 24 formant un second élément de mesure pour mesurer une valeur d'écartement entre le plateau fémoral 31 et le plateau tibial 21.

La base 20 présente également deux goupilles 25, en saillie de la face frontale 201, prévues pour permettre un montage amovible d'un appareil de mesure 9 (décrit ultérieurement) sur la base 20.

La base 20 présente également deux trous de maintien 26, débouchant dans la face frontale 201, prévus pour permettre un montage amovible d'un élément de guidage 6 (décrit ultérieurement) sur la base 20. L'un des deux trous de maintien 26 est de section oblongue tandis que l'autre est de section circulaire.

La base 20 présente également un orifice de réglage 27, débouchant dans la face frontale 201 et débouchant tangentiellement dans l'orifice de guidage 22, cet orifice de réglage 27 s'étendant selon une direction parallèle à l'axe antéro-postérieur AP (décrit ultérieurement). Cet orifice de réglage 27 est prévu pour accueillir un pignon 50 d'un système de réglage 5 (décrit ultérieurement).

La base 20 présente également un premier doigt 28, en saillie de la face frontale 201, et disposé au-dessus de l'orifice de réglage 27, ce premier doigt 28 étant prévu pour recevoir à pivot pour une gâchette 54 d'un mécanisme de verrouillage réversible (décrit ultérieurement).

La base 20 présente également un second doigt 29, en saillie de la face frontale 201, et disposé au-dessus des deux goupilles 25, ce second doigt 29 étant prévu pour participer au maintien de la gâchette 54 précitée en position de verrouillage, comme décrit ultérieurement.

En référence aux figures 1, 3, 4, 11 et 12, l'élément fémoral 3 est monté pivotant sur l'élément mobile 4 selon un axe antéro-postérieur AP, de sorte que le plateau fémoral 31 est pivotant autour de cet axe antéro-postérieur AP relativement au plateau tibial 21 qui est statique.

En référence à la figure 2, l'élément fémoral 3 est formé d'une pièce monobloc et comprend principalement le plateau fémoral 31, au milieu duquel fait saillie un arbre de pivot 30 s'étendant selon l'axe antéro-postérieur AP ; cet arbre de pivot 30 formant le pivot pour l'élément fémoral 3.

Par ailleurs, l'élément fémoral 3 comprend un bras fémoral 32 solidaire du plateau fémoral 31, où ce bras fémoral 32 s'étend sensiblement dans le plan du plateau fémoral 31 et fait saillie d'un bord du plateau fémoral 31. Ce bras fémoral 32 se termine par une extrémité libre qui se présente sous la forme d'une mâchoire 33 comprenant deux mors 34, 35 en vis-à-vis mobiles entre une position serrée et une position desserrée. Les mors 34, 35 comprennent un mors interne 34 et un mors externe 35, le mors interne 34 étant situé plus proche du plateau fémoral 31 que le mors externe 35.

Plus précisément, la mâchoire 33 est formée d'une glissière fendue dans laquelle est prévue une fente 330 entre les deux mors 34, 35, cette fente 330 s'étendant dans un plan orthogonal à un axe transversal AT qui est luimême orthogonal à l'axe antéro-postérieur AP et à l'axe longitudinal AL.

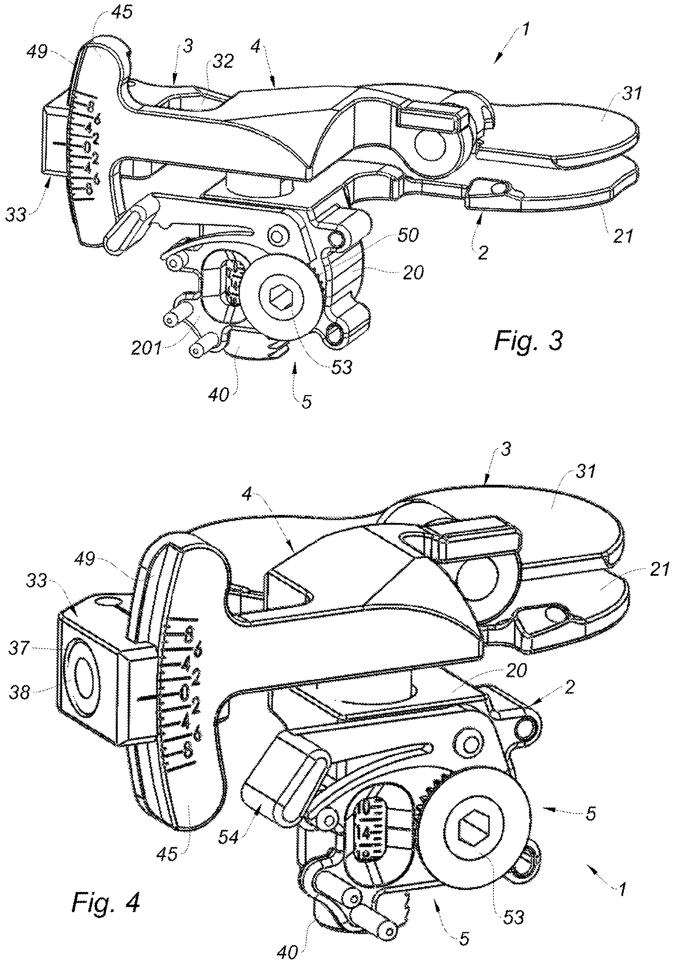

Il est par ailleurs prévu un orifice de serrage 37 qui traverse les deux mors 34, 35, et qui traverse donc également la fente 330, en s'étendant selon l'axe transversal AT. Cet orifice de serrage 37 présente une portion lisse dans le mors externe 35, et une portion taraudée dans le mors interne 34. Cet orifice de serrage 37 débouche dans une face 350 du mors externe 35 qui est orthogonal à l'axe transversal AT afin de permettre l'insertion d'une vis de serrage 38 (visible sur les figures 4, 11 et 12).

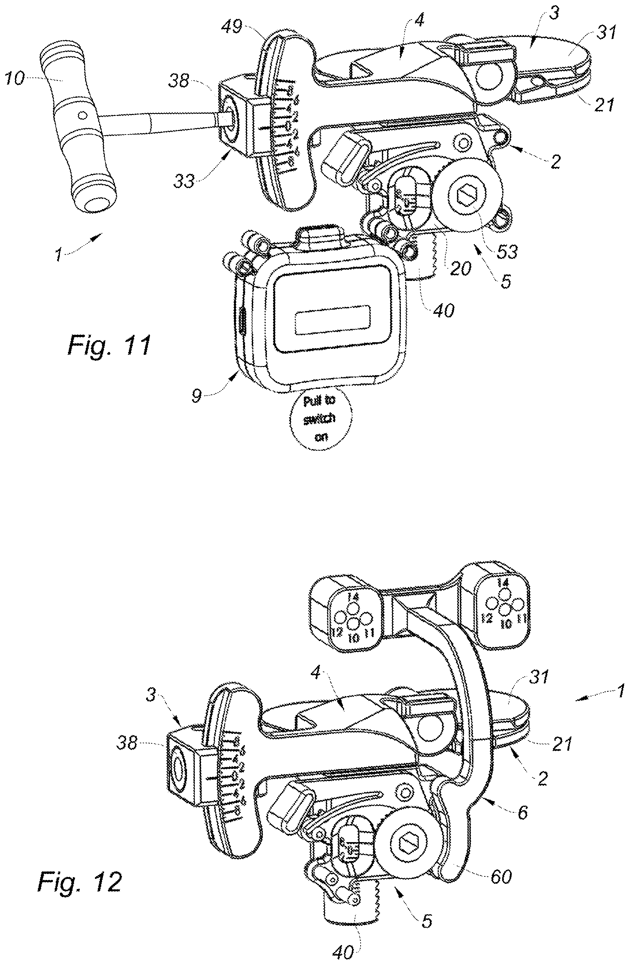

La vis de serrage 38 présente une tige filetée en prise dans la portion taraudée de l'orifice de serrage 37, et elle présente une tête munie d'une empreinte propre à coopérer avec un tournevis de serrage 10 (visible sur la figure 11) ; cette tête de la vis de serrage 38 venant en butée contre un épaulement interne prévu dans la portion lisse de l'orifice de serrage 37.

Ainsi, la vis de serrage 38 forme un élément de serrage coopérant avec la mâchoire 33 pour déplacer les mors 34, 35 entre une position serrée (où les mors 34, 35 sont rapprochés et la fente 330 est réduite) et une position desserrée (où les mors 34, 35 sont écartés et la fente 330 est agrandie comparativement à la position serrée). Ainsi, le vissage de la vis de serrage 38 déplace les mors 34, 35 vers la position serrée et le dévissage de la vis de serrage 38 déplace les mors 34, 35 vers la position desserrée.

Le mors externe 35 présente une encoche 351 tournée vers le mors interne 34, et le mors interne 34 présente un épaulement 341 tourné vers le mors externe 35 ; l'encoche 351 et l'épaulement 341 étant ainsi en vis-à-vis.

Il est également prévu un repère de référence 36 ménagé sur le mors externe 35, ce repère de référence 36 formant un second élément de repérage pour mesurer une valeur d'angle de pivotement du plateau fémoral 31 relativement au plateau tibial 21.

L'élément fémoral 3 présente également un plot central 39, en saillie d'une face supérieure du plateau fémoral 31 et situé dans un plan médian du plateau fémoral 31 incluant l'axe antéro-postérieur AP ; cette face supérieure étant opposée à une face inférieure qui fait face au plateau tibial 21. L'arbre de pivot 30 prolonge ce plot central 39.

Ce plot central 39 présente, de part et d'autre du plan médian, deux encoches 390 opposées dont les formes définissent :
- des détrompeurs inférieurs pour permettre de positionner de manière univoque un capteur de force 8 (décrit ultérieurement) sur la face supérieure du plateau fémoral 31 ; et
- des détrompeurs supérieurs pour permettre de positionner de manière univoque une cale de compensation 7 (décrite ultérieurement) sur la face supérieure du plateau fémoral 31 ou sur le capteur de force 8 lorsque présent.

En référence à la figure 5, l'élément mobile 4 est formé d'une pièce monobloc et comprend principalement le guide 40 s'étendant selon l'axe longitudinal AL et surmonté d'un corps 41 s'étendant transversalement de part et d'autre de l'axe longitudinal AL.

Le guide 40 se présente sous la forme d'une tige munie d'une crémaillère 42 formée d'une série de dents régulièrement réparties selon l'axe longitudinal AL sur une tranche de la tige, où ce guide 40 est monté coulissant à l'intérieur de l'orifice de guidage 22 prévu dans la base 20 de l'élément tibial statique 2. La crémaillère 42 présente ainsi un pas qui correspond à l'écartement entre deux dents successives.

Le guide 40 présente par ailleurs une clavette (non visible) prévue pour s'engager dans la rainure 220 de l'orifice de guidage 22 pour empêcher la rotation du guide 40 à l'intérieur de l'orifice de guidage 22.

Le système de réglage 5 comprend un pignon 50 qui est monté pivotant sur la base 20 de l'élément tibial statique 2 selon une direction parallèle à l'axe antéro-postérieur AP, et qui est plus précisément monté pivotant à l'intérieur de l'orifice de réglage 27.

Comme visible sur la figure 7, ce pignon 50 présente :
- une première portion de pignon 51 formant une partie d'un mécanisme de verrouillage réversible, où cette première portion de pignon 51 se présente sous la forme d'une roue crantée comprenant des crans unidirectionnels (ou asymétriques) en périphérie, et cette première portion de pignon 51 dépasse de la base 20 au niveau de sa face frontale 201 ;
- une seconde portion de pignon 52 prévue pour être en engrènement avec la crémaillère ou série de dents 42 du guide 40, où cette seconde portion de pignon 52 se présente sous la forme d'une roue crantée comprenant des crans bidirectionnels (ou symétriques) en périphérie.

Le pignon 50 est solidaire en rotation d'une vis de réglage 53 munie d'une empreinte propre à coopérer avec le tournevis de serrage 10 précité (visible sur la figure 11).

Ainsi, le vissage de la vis de réglage 53 déplace en rotation le pignon 50 dans un premier sens de rotation S1, ce qui entraîne le guide 40 en translation vers le haut pour déplacer le plateau fémoral 31 dans le sens d'un écartement vis-à-vis du plateau tibial 21.

A l'inverse, le dévissage de la vis de réglage 53 déplace en rotation le pignon 50 dans un second sens de rotation S2 (opposé au premier sens de rotation S1), ce qui entraîne le guide 40 en translation vers le bas pour déplacer le plateau fémoral 31 dans le sens d'un rapprochement vis-à-vis du plateau tibial 21, sous réserve que la gâchette 54 soit en position de déverrouillage comme décrit ci-après.

Par ailleurs, le guide 40 présente en outre une échelle de graduations 48 réparties le long de l'axe longitudinal AL ; cette échelle de graduations 48 formant un premier élément de mesure pour mesurer une valeur d'écartement entre le plateau fémoral 31 et le plateau tibial 21. En effet, cette échelle de graduations 48 est visible à travers la fenêtre 23 prévue sur la face frontale 201 de la base 20, et le repère 24 prévu sur le côté de la fenêtre 23 en correspondance visuelle avec l'échelle de graduations 48, de manière à mesurer une valeur d'écartement selon l'axe longitudinal AL entre le plateau fémoral 31 et le plateau tibial 21. A titre, d'exemple, l'échelle de graduations 48 est graduée tous les 1 millimètres.

Le système de réglage 5 comprend un mécanisme de verrouillage réversible accouplé entre la base 20 de l'élément tibial statique 2 et le pignon 50 pour verrouiller de manière réversible la rotation du pignon 50 et par conséquent la mobilité du guide 40 relativement à la base, afin de fixer l'écartement entre le plateau fémoral 31 et le plateau tibial 21.

Dans l'exemple illustré, le mécanisme de verrouillage réversible comprend une gâchette 54 montée pivotante sur la base 20 selon une direction parallèle à l'axe antéro-postérieur AP, et plus précisément la gâchette 54 est montée pivotante sur le premier doigt 28 prévu en saillie sur la face frontale 201 de la base 20.

La gâchette 54 est mobile entre :
- une position de verrouillage (visible en figure 7) dans laquelle la gâchette 54 coopère avec le pignon 50 pour le verrouiller en rotation de manière unidirectionnel selon le second sens de rotation S2 (correspondant à une réduction de l'écartement entre le plateau fémoral 31 et le plateau tibial 21), tout en autorisant la rotation du pignon 50 selon le premier sens de rotation S1 (correspondant à un accroissement de l'écartement entre le plateau fémoral 31 et le plateau tibial 21) ; et
- une position de déverrouillage dans laquelle la gâchette 54 ne coopère pas avec le pignon 50 qui est alors libre de pivoter dans les deux sens de rotation S1, S2.

Plus précisément, cette gâchette 54 est munie d'un bras 55 pivotant muni d'une première extrémité sur laquelle est prévu un cliquet 56 propre à se bloquer dans les crans unidirectionnels de la première portion de pignon 51 du pignon 50 dans la position de verrouillage, pour :
- procurer un blocage dans ces crans unidirectionnels pour interdire la rotation du pignon 50 selon le second sens de rotation S2 ;
- tout en autorisant la rotation du pignon 50 selon le premier sens de rotation S1.

Le bras 55 de la gâchette 54 est également muni d'une seconde extrémité, opposée à la première extrémité, sur laquelle est prévue une surface d'appui 57 permettant au chirurgien d'appuyer dessus pour faire pivoter le bras 55 dans le sens d'un désaccouplement du cliquet 56 avec les crans unidirectionnels de la première portion de pignon 51 du pignon 50, ce qui procudre le déverrouillage de la gâchette 54. Ainsi, en appuyant sur ce support de pression 57, la gâchette 54 se désaccouple du pignon 50 (position de déverrouillage) qui est ainsi autorisé à tourner dans les deux sens de rotation S1, S2.

Cette gâchette 54 est également munie d'un organe élastique de rappel 58 sollicitant le bras 55 vers la position de verrouillage, autrement dit vers une position de blocage (ou d'accouplement) du cliquet 56 dans les crans unidirectionnels de la première portion de pignon 51 du pignon 50. Cet organe élastique de rappel 58 se présente sous la forme d'un levier solidaire du cliquet et ayant une extrémité libre qui prend appui sur le second doigt 29.

Le corps 41 de l'élément mobile 4, qui est fixé solidairement à une extrémité supérieure du guide 40, présente deux bras 43, 44 s'étendant transversalement à l'axe longitudinal AL, de part et d'autre de cet axe longitudinal AL, avec :
- un bras transversal 43 se terminant par une extrémité libre munie d'une arche 45, de sorte que le bras transversal 43 présente une forme générale en « T » ;
- un bras de pivot 44 se terminant par une extrémité libre munie d'un trou de pivot 47 propre à recevoir à pivot l'arbre de pivot 30 l'élément fémoral 3, de sorte que l'élément fémoral 3 est pivotant selon l'axe antéro-postérieur AP sur ce bras de pivot 44.

L'arche 45 est munie d'une échelle de graduations 46 réparties autour de l'axe antéro-postérieur AP, cette échelle de graduations 46 formant un premier élément de repérage pour mesurer une valeur d'angle de pivotement du plateau fémoral 31 relativement au plateau tibial 21. En effet, le repère de référence 36 ménagé sur le mors externe 35 de l'élément fémoral 3 vient en correspondance visuelle avec l'échelle de graduations 46, de manière à mesurer une valeur d'angle de pivotement du plateau fémoral 31 relativement au plateau tibial 21. A titre d'exemple, l'échelle de graduations 46 est graduée tous les 1 degré, entre - 10 degrés et + 10 degrés, l'angle nul ou 0 degré correspondant à un parallélisme entre le plateau fémoral 31 et le plateau tibial 21.

L'arche 45 présente un bord externe arqué, selon une courbure sensiblement centrée sur l'axe antéro-postérieur AP, et dans lequel est prévu un rail 49 le long duquel est disposée l'échelle de graduations 46.

Ce rail 49 est donc prévu sur l'extrémité libre du bras transversal 43 et ce rail 49 est prévu pour s'engager à l'intérieur de la mâchoire 33 entre les deux mors 34, 35, et plus précisément ce rail 49 est intercalé entre :
- l'encoche 351 prévue sur le mors externe 35, le rail 49 étant alors bloqué par cette encoche 351 ; et
- l'épaulement 341 prévu sur le mors interne 34.

Ainsi, l'instrument chirurgical 1 comprend en outre un système de blocage réversible accouplé entre le bras transversal 43 de l'élément mobile 4 et le bras fémoral 32 de l'élément fémoral 3 pour immobiliser de manière réversible le pivotement de l'élément fémoral 3 par rapport à l'élément mobile 4 afin de fixer l'angle de pivotement du plateau fémoral 31 relativement au plateau tibial 21 à différentes valeurs d'angle de pivotement.

Ce système de blocage réversible comprend :
- la mâchoire 33 comprenant les deux mors 34, 35 en vis-à-vis mobiles entre une position serrée et une position desserrée ;
- le rail 49 engagé à l'intérieur de la mâchoire 33 entre les deux mors 34, 35 ;
- la vis de serrage 38 qui forme un élément de serrage coopérant avec la mâchoire 33 pour déplacer les mors 34, 35 entre la position serrée et la position desserrée.

Dans la position serrée, le rail 49 est coincé entre les deux mors 34, 35 et il est donc bloqué, de sorte que l'élément fémoral 3 est immobilisé dans son pivotement par rapport à l'élément mobile 4.

Dans la position desserrée, le rail 49 est libre de se déplacer entre les deux mors 34, 35 et il est donc libre de pivoter autour de l'axe antéro-postérieur AP, de sorte que l'élément fémoral 3 est autorisé à pivoter par rapport à l'élément mobile 4. Le chirurgien peut se servir du tournevis de serrage 10 comme d'un bras de levier, une fois enclenché avec la vis de serrage 38, afin de faire pivoter l'élément fémoral 3 par rapport à l'élément mobile 4 lorsque les mors 34, 35 sont en position desserrée, puis lorsqu'il estime que l'angle de pivotement lui convient, il suffit alors de visser la vis de serrage 38 et ainsi de resserrer la mâchoire 33 pour être position serrée.

Ainsi, ce tournevis de serrage 10 offre une double fonction :
- une fonction bras de levier pour orienter en pivotement le plateau fémoral 31 autour de l'axe antéro-postérieur AP ;
- une fonction de serrage pour contrôler la fermeture de la mâchoire 33.

Cet instrument chirurgical 1 peut être utilisé avec un capteur de force 8 adapté pour être placé de manière amovible sur la face supérieure du plateau fémoral 31, afin de détecter une ou plusieurs forces exercées par les condyles du fémur sur le plateau fémoral 31.

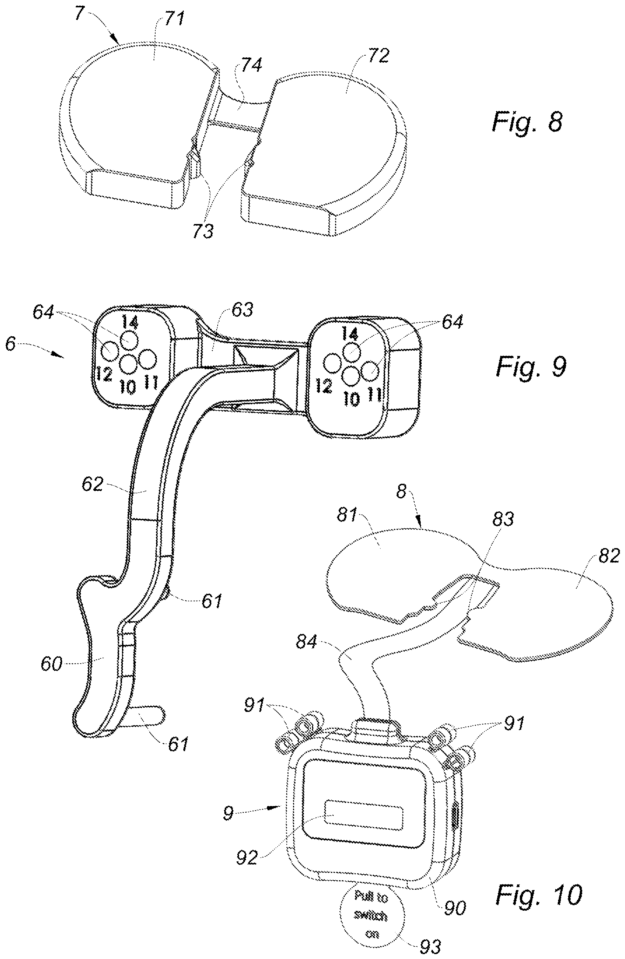

Comme visible sur la figure 10, ce capteur de force 8 comprend un détecteur médial 81 prévu pour détecter une force exercée par le condyle médial du fémur sur le plateau fémoral 31, et un détecteur latéral 82 prévu pour détecter une force exercée par le condyle latéral du fémur sur le plateau fémoral 31. La situation peut être inversée selon qu'il s'agit du genou droit ou du genou gauche.

Ce capteur de force 8 est muni de deux ergots 83 en vis-à-vis configurés pour s'engager dans les encoches 390 du plot central 39 de l'élément fémoral 3, afin de s'accoupler avec les détrompeurs inférieurs pour permettre de positionner de manière univoque ce capteur de force 8.

Ce capteur de force 8 est raccordé à un appareil de mesure 9, par une voie non filaire ou par une voie filaire comme dans l'exemple illustré, cette voie filaire se présentant sous la forme d'une nappe de connexion 84. Il est à noter que le bras de pivot 44 peut présenter, au-dessus du trou de pivot 47, une plateforme 440 servant de guide ou support afin d'aider au positionnement de la nappe de connexion 84.

Comme visible sur la figure 10, cet appareil de mesure 9 comprend un boîtier 90 à l'intérieur duquel est prévue une batterie électrique et une unité de contrôle, non visible et comprenant par exemple une puce électronique, un processeur, un contrôleur et/ou une carte électronique ; cette unité de contrôle étant prévue pour recevoir les mesures effectuées par le capteur de force 8.

Par ailleurs, le boîtier 90 présente sur ses côtés, à droite et à gauche, deux fûts de maintien 91 annulaires propres à recevoir intérieurement les deux goupilles 25 afin de pouvoir monter de manière amovible l'appareil de mesure 9 sur la base 20 de l'élément tibial statique 2. Il y a deux fûts de maintien 91, à droite et à gauche, pour s'adapter à la fois à un instrument chirurgical 1 propre à un genou droit et à un instrument chirurgical 1 propre à un genou gauche.

Cet appareil de mesure 9 est également muni d'un afficheur 92 en façade, procurant un affichage des mesures effectuées par le capteur de force 9. Une languette de mise en fonctionnement 93 est également prévue pour mettre en contact la batterie électrique et un contacteur embarqué (non visible) pour mettre le capteur de force 8 en fonctionnement.

Cet instrument chirurgical 1 peut être utilisé avec une cale de compensation 7 adaptée pour être placée de manière amovible sur la face supérieure du plateau fémoral 31, et notamment sur le capteur de force 8 si ce dernier est présent.

Comme visible sur la figure 8, cette cale de compensation 7 comprend un compartiment médial 71 prévu pour un appui du condyle distal médial du fémur, et un compartiment latéral 72 prévu pour un appui du condyle distal latéral du fémur. Le compartiment médial 71 et le compartiment latéral 72 sont reliés par un pont central 74. La situation peut être inversée selon qu'il s'agit du genou droit ou du genou gauche.

Cette cale de compensation 7 est munie de deux ergots 73 en vis-à-vis configurés pour s'engager dans les encoches 390 du plot central 39 de l'élément fémoral 3, afin de s'accoupler avec les détrompeurs supérieurs (au-dessus des détrompeurs inférieurs) pour permettre de positionner de manière univoque cette cale de compensation 7.

Cet instrument chirurgical 1 peut aussi être utilisé avec un élément de guidage 6 formant un positionneur pour des clous qui serviront ensuite à fixer un ou des guides de coupe fémorale.

Comme visible sur la figure 9, cet élément de guidage 6 est formé d'une pièce monobloc comprenant une embase 60 munie de deux goupilles de maintien 61 propres à s'engager dans les deux trous de maintien 26 prévus sur la base 20 de l'élément tibial statique 2, afin de monter de manière amovible cet élément de guidage 6 sur la base 20.

Cet élément de guidage 6 comprend un bras 62 prolongeant l'embase 60 et se terminant par une extrémité solidaire d'un support 63 dans lequel sont prévus plusieurs orifices de guidage 64, à droite et à gauche, pour positionner ou guider des perçages dans le fémur ; de tels perçages pouvant servir ensuite pour fixer le ou les guides de coupe fémorale.

Les orifices de guidage 64 forment ainsi des canons de perçage correspondents à différentes épaisseurs d'insert et vont par paires parallèles au plan d'appui du plateau tibial 21 sur l'extrémité réséquée du tibia.

Le parallélisme de ces orifices de guidage 64 et la maîtrise de leur positionnement via les goupilles de maintien 61 permettent d'obtenir un équilibrage ligamentaire en fin de chirurgie équivalent à l'équilibrage ligamentaire obtenu lors de l'utilisation de l'instrument chirurgical 1, à quelques imprécisions de coupes et de positionnement d'implants près.

La suite de la description porte sur l'utilisation de l'instrument chirurgical 1 par un chirurgien.

Après avoir effectué la coupe tibiale et la coupe distale fémorale, et après avoir placé la jambe en extension, le chirurgien insère dans l'articulation l'instrument chirurgical 1, la cale de compensation 7 et le capteur de force 8. La rotule est remise en place.

La rotation du plateau fémoral 21 est éventuellement verrouillée sur une valeur d'angle de pivotement de 0 degré à l'aide de la vis de verrouillage 38 et du tournevis de serrage 10. L'afficheur 92 de l'appareil de mesure 9 indique au chirurgien une évaluation objective de la tension ligamentaire.

L'articulation est ensuite mise en tension avec le tournevis de serrage 10 inséré dans la vis de réglage 53 couplée au pignon 50 jusqu'à obtenir une tension que le chirurgien estime satisfaisante. La valeur de l'écartement entre le plateau fémoral 31 et le plateau tibial 21 (ou valeur de distraction) est indiquée par le le repère 24 en correspondance visuelle avec l'échelle de graduations 48, et cette valeur correspond à l'épaisseur de l'insert qu'il faudra utiliser lors de la mise en place des implants définitifs.

Après avoir placé la jambe en flexion, le chirurgien insère dans l'articulation l'instrument chirurgical 1 et le capteur de force 8, et il remet la rotule en place.

La rotation du plateau fémoral 21 est déverrouillée au moyen du tournevis de serrage 10 engagé dans la vis de verrouillage 38, afin que le plateau fémoral 21 pivote librement autour de l'axe antéro-postérieur AP. L'afficheur 92 de l'appareil de mesure 9 indique une évaluation objective de la tension ligamentaire.

L'articulation est ensuite mise en tension avec le tournevis de serrage 10 inséré dans la vis de réglage 53 couplée au pignon 50 jusqu'à obtenir une tension que le chirurgien estime satisfaisante. Lors de la mise en tension, le plateau fémoral 21 pivote librement autour de l'axe antéro-postérieur AP ce qui tend à équilibrer les forces. Le chirurgien peut se satisfaire de l'équilibre obtenu mécaniquement ou ajuster l'équilibrage en se servant de l'effet bras de levier offert par le tournevis de serrage engagé dans la vis de verrouillage 38, l'ajustement ainsi obtenu est figé en vissant la vis de verrouillage 38 jusqu'à verrouiller la rotation du plateau fémoral 31.

L'élément de guidage 6 est alors monté sur la base 20 de l'élément tibial statique 2, et le chirurgien se sert de l'épaisseur d'insert obtenue plus tôt pour identifier les orifices de guidage 64 dans lesquels effectuer les perçages pour ensuite visser deux broches de fixation du guide de coupe fémorale.

L'instrument chirurgical 1, le capteur de force 8 et l'élément de guidage 6 sont ensuite extraits de l'articulation, et le guide de coupe fémorale est mis en place sur les deux broches précédemment vissées. Ainsi, les coupes fémorales peuvent être effectuées et les implants peuvent être mis en place. A quelques imprécisions de coupes et de positionnement d'implant près, l'équilibrage ligamentaire est à la fin reproduit par les implants.

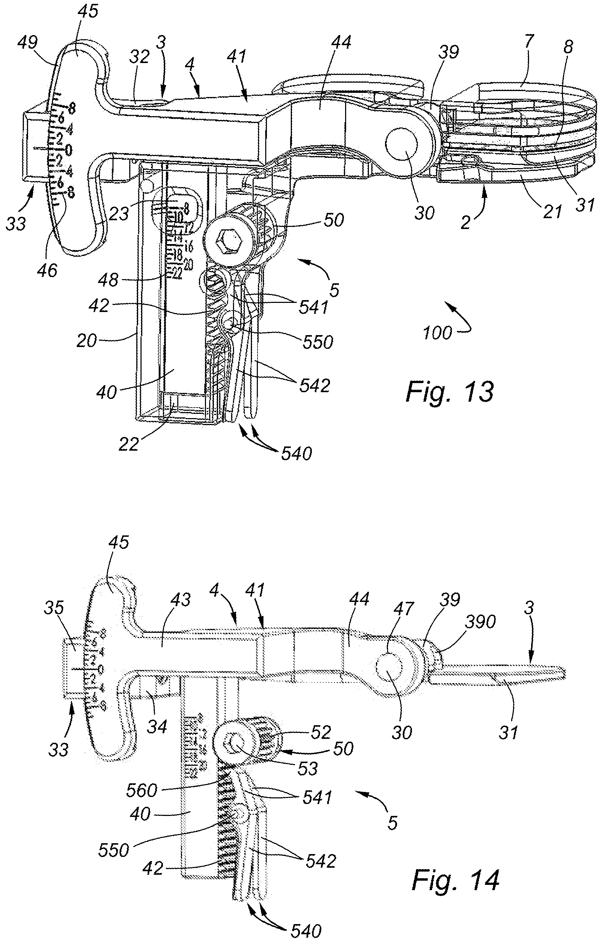

Les figures 13 à 15 illustrent un instrument chirurgical 100 selon un second mode de réalisation, qui est similaire à l'instrument chirurgical 1 décrit précédemment, dans le sens où il comporte les mêmes éléments principaux qui sont :
- l'élément tibial statique 2 avec notamment sa base 20 et son plateau tibial 21, son orifice de guidage 22 et sa fenêtre 23 ;
- l'élément fémoral 3 avec son plateau fémoral 31 et son bras fémoral 32, son plot central 39 avec ses encoches 390 et son arbre de pivot 30 ;
- l'élément mobile 4 avec son guide 40 et sa crémaillère 42, son échelle de graduations 48, son bras transversal 43 avec son arche 45 et son bras de pivot 44 avec son trou de pivot 47 ;
- le système de blocage réversible avec sa mâchoire 33 et ses deux mors 34, 35, son rail 49 et sa vis de serrage 38.

Cependant, cet instrument chirurgical 100 se distingue principalement de l'instrument chirurgical 1, dans la forme du mécanisme de verrouillage réversible configuré pour verrouiller de manière réversible la mobilité du guide 40 relativement à la base 20, afin de fixer l'écartement entre le plateau fémoral 31 et le plateau tibial 21.

Pour rappel, dans l'instrument chirurgical 1, le mécanisme de verrouillage réversible comprend une gâchette 54 montée mobile sur la base 20 de l'élément tibial statique 2 afin de coopérer avec le pignon 50 pour le verrouiller en rotation. Dans cet instrument chirurgical 100, le mécanisme de verrouillage réversible comprend deux pênes 540 montés tous les deux mobiles sur la base 20 de l'élément tibial statique 2 afin de coopérer avec la crémaillère 42 (et non pas avec le pignon 50) pour verrouiller directement et de manière réversible la mobilité de cette crémaillère 42, et donc du guide 40, en translation.

Chaque pêne 540 est monté pivotant sur la base 20 selon une direction parallèle à l'axe antéro-postérieur AP (et donc orthogonal à l'axe longitudinal AL), et plus spécifiquement sur un axe physique 550 porté par la base 20, où cet axe physique 550 est de préférence commun aux deux pênes 540. Ainsi, chaque pêne 540 est mobile entre :
- une position de verrouillage dans laquelle le pêne 540 coopère avec la crémaillère 42, et plus spécifiquement est en engagement entre deux dents de la crémaillère 42, pour la verrouiller en translation ; et
- une position de déverrouillage dans laquelle le pêne 540 ne coopère pas avec les dents de la crémaillère 42, et plus spécifiquement est écarté des dents suffisamment pour autoriser la crémaillère 42 et donc aussi le guide 40 à translater.

Plus précisément, chaque pêne 540 comprend un bras pivotant autour de l'axe physique 550 et muni, de part et d'autre de l'axe physique 550, de deux portions opposées qui sont :
- une première portion 541 se terminant par une première extrémité sur laquelle est prévu un cliquet 560 propre à se bloquer entre les dents de la crémaillère 42 pour procurer un blocage ; et
- une seconde portion 542 accessible hors de la base 20 et sur laquelle peut agir le chirurgien, en appuyant dessus, dans le sens d'un désaccouplement du cliquet 560 avec les dents de la crémaillère 42, ce qui procudre le déverrouillage du pêne 540.

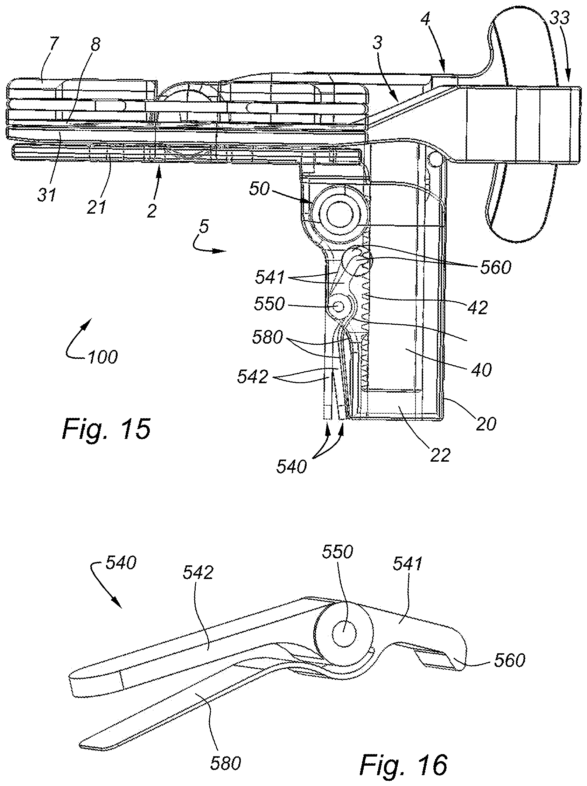

Il est également prévu au moins un organe élastique de rappel 580 sollicitant chaque pêne 540 vers sa position de verrouillage, autrement dit vers une position de blocage (ou d'accouplement) du cliquet 560 dans les dents de la crémaillère 42. A titre d'exemple et en référence à la figure 16, chaque pêne 540 est solidaire d'un organe élastique de rappel 580 qui lui est propre, et cet organe élastique de rappel 580 se présente sous la forme d'une lame élastique solidaire du pêne 540 et qui présente une partie libre s'étendant le long de la seconde portion 542, où cette partie libre est propre à venir en appui contre la crémaillère 42 située en face afin d'exercer un effort de rappel qui tend à éloigner la seconde portion 542 de la crémaillère 42 et donc à rapprocher le cliquet 560 de la crémaillère 42.

Ainsi, pour autoriser la crémaillère 42 et donc aussi le guide 40 à translater, le chirurgien doit appuyer sur les secondes portions 542 des deux pênes 540 disposés côte-à-côte, à l'encontre des organes élastiques de rappel 580.

Par ailleurs, ces pênes 540 sont décalés l'un de l'autre selon l'axe longitudinal AL d'un écart correspondant à la moitié d'un pas de la crémaillère 42, de sorte que lorsqu'un pêne 540 est en position de verrouillage entre deux dents de la crémaillère 42 alors l'autre pêne 540 est en position de déverrouillage en étant au contact d'un sommet d'une dent de la crémaillère 42.

Il est à noter enfin que, dans cet instrument chirurgical 100, le pignon 50 présente uniquement la seconde portion de pignon 52 qui est en engrènement avec la crémaillère ou série de dents 42 du guide 40, où cette seconde portion de pignon 52 se présente sous la forme d'une roue crantée comprenant des crans bidirectionnels (ou symétriques) en périphérie. En effet, la première portion 51 décrite précédemment est inutile dans cet instrument chirurgical 100, dans la mesure où les pênes 540 viennent en prise directement avec la crémaillère 42.

## Revendications

1. Instrument chirurgical (1 ; 100) pour un équilibrage du genou dans le cadre d'une préparation à la pose chirurgicale d'une prothèse totale de genou, ledit instrument chirurgical (1) comprenant :
- un élément tibial statique (2) incluant une base (20) solidaire d'un plateau tibial (21) prévu pour venir en appui sur une extrémité proximale reséquée du tibia ;
- un élément fémoral (3) incluant un plateau fémoral (31) prévu pour venir en appui sur les condyles distaux ou postérieurs du fémur ou sur une extrémité distale reséquée du fémur ou sur une cale de compensation (7) ;
- un élément mobile (4) incluant un guide (40) monté mobile en translation sur la base (20) de l'élément tibial statique (2) selon un axe longitudinal (AL), et dans lequel l'élément fémoral (3) est monté pivotant sur ledit élément mobile (4) selon un axe antéro-postérieur (AP), de sorte que le plateau fémoral (31) est pivotant autour dudit axe antéro-postérieur (AP) relativement au plateau tibial (21) qui est statique ;
- un système de réglage (5) accouplé entre le guide (40) de l'élément mobile (4) et la base (20) de l'élément tibial statique (2) pour un réglage de la mobilité dudit guide (40) relativement à ladite base (20) afin de procurer une fonction de distraction en permettant un réglage de l'écartement entre le plateau fémoral (31) et le plateau tibial (21) ;
dans lequel l'élément mobile (4) comprend un bras transversal (43) solidaire du guide (40), et l'élément fémoral (3) comprend un bras fémoral (32) solidaire du plateau fémoral (31) ;
et dans lequel l'instrument chirurgical (1 ; 100) comprend en outre un système de blocage réversible accouplé entre le bras transversal (43) de l'élément mobile (4) et le bras fémoral (32) de l'élément fémoral (3) pour immobiliser de manière réversible le pivotement de l'élément fémoral (3) par rapport à l'élément mobile (4) afin de fixer l'angle de pivotement du plateau fémoral (31) relativement au plateau tibial (21) à différentes valeurs d'angle de pivotement,
ledit instrument chirurgical (1 ; 100) étant **caractérisé en ce que** le système de blocage réversible comprend :
- une mâchoire (33) qui est prévue sur une extrémité libre du bras fémoral (32), et qui comprend deux mors (34, 35) en vis-à-vis mobiles entre une position serrée et une position desserrée ;
- un rail (49) qui est prévu sur une extrémité libre du bras transversal (43) de l'élément mobile (4) et qui est engagé à l'intérieur de la mâchoire (33) entre les deux mors (34, 35) ;
- un élément de serrage (38) coopérant avec la mâchoire (33) pour déplacer les mors (34, 35) entre la position serrée et la position desserrée, où dans la position serrée l'élément fémoral (3) est immobilisé dans son pivotement par rapport à l'élément mobile (4), et où dans la position desserrée l'élément fémoral (3) est autorisé à pivoter par rapport à l'élément mobile (4) ;
**en ce que** ledit élément de serrage comprend une vis de serrage (38) vissée dans un orifice de serrage (37) qui traverse les deux mors (34, 35), de sorte que le vissage de la vis de serrage (38) déplace les mors (34, 35) vers la position serrée et le dévissage de la vis de serrage (38) déplace les mors (34, 35) vers la position desserrée ;
et **en ce que** la vis de serrage (38) et l'orifice de serrage (37) s'étendent selon un axe transversal (AT) orthogonal à l'axe antéro-postérieur (AP).

2. Instrument chirurgical (1 ; 100) selon la revendication 1, dans lequel le bras transversal (43) de l'élément mobile (4) se termine par une extrémité libre munie d'un premier élément de repérage (46), et le bras fémoral (32) de l'élément fémoral (3) se termine par une extrémité libre munie d'un second élément de repérage (36), où le premier élément de repérage (46) et le second élément de repérage (36) comprennent une échelle de graduations réparties autour de l'axe antéro-postérieur (AP) et un repère de référence en correspondance visuelle avec l'échelle de graduations, de manière à mesurer une valeur d'angle de pivotement du plateau fémoral (31) relativement au plateau tibial (21)

3. Instrument chirurgical (1 ; 100) selon l'une quelconque des revendications 1 et 2, dans lequel la mâchoire (33) est formée d'une glissière fendue dans laquelle est prévue une fente (330) entre les deux mors (34, 35).

4. Instrument chirurgical (1 ; 100) selon l'une quelconque des revendications précédentes, dans lequel le guide (40) comprend une crémaillère (42) formée d'une série de dents, et le système de réglage (5) comprend un pignon (50) qui est monté pivotant sur la base (20) de l'élément tibial statique (2) et qui est en engrènement avec la crémaillère (42).

5. Instrument chirurgical (1) selon la revendication 4, dans lequel le système de réglage (5) comprend un mécanisme de verrouillage réversible accouplé entre la base (20) de l'élément tibial statique (2) et le pignon (50) pour verrouiller de manière réversible la rotation du pignon (50) et par conséquent la mobilité du guide (40) relativement à la base (20), afin de fixer l'écartement entre le plateau fémoral (31) et le plateau tibial (21).

6. Instrument chirurgical (1) selon la revendication 5, dans lequel le mécanisme de verrouillage réversible comprend une gâchette (54) montée mobile sur la base (20) de l'élément tibial statique (2) entre une position de verrouillage dans laquelle la gâchette (54) coopère avec le pignon (50) pour le verrouiller en rotation, et une position de déverrouillage dans laquelle la gâchette (54) ne coopère pas avec le pignon (50) qui est libre de pivoter.

7. Instrument chirurgical (100) selon la revendication 4, dans lequel le système de réglage (5) comprend un mécanisme de verrouillage réversible accouplé entre la base (20) de l'élément tibial statique (2) et la crémaillère (42) pour verrouiller directement et de manière réversible la mobilité du guide (40) relativement à la base (20), afin de fixer l'écartement entre le plateau fémoral (31) et le plateau tibial (21).

8. Instrument chirurgical (100) selon la revendication 7, dans lequel le mécanisme de verrouillage réversible comprend au moins un pêne (540) monté pivotant sur la base (20) entre une position de verrouillage dans laquelle le pêne (540) est en engagement entre deux dents de la crémaillère (42) pour la verrouiller en translation, et une position de déverrouillage dans laquelle le pêne (540) ne coopère pas avec les dents de la crémaillère (42) pour l'autoriser à translater.

9. Instrument chirurgical (100) selon la revendication 8, dans lequel le mécanisme de verrouillage réversible comprend deux pênes (540) montés pivotant sur la base entre une position de verrouillage et une position de déverrouillage,
et dans lequel lesdits pênes (540) sont décalés l'un de l'autre selon l'axe longitudinal (AL) d'un écart correspondant à la moitié d'un pas de la crémaillère (42), le pas de la crémaillère (42) correspondant à l'écartement entre deux dents successives, de sorte que lorsqu'un pêne (540) est en position de verrouillage entre deux dents de la crémaillère (42) alors l'autre pêne (540) est en position de déverrouillage en étant au contact d'un sommet d'une dent de la crémaillère (42).

10. Instrument chirurgical (1 ; 100) selon l'une quelconque des revendications 4 à 9, dans lequel :
- le guide (40) est muni d'un premier élément de mesure (48) ;
- la base (20) de l'élément tibial statique (2) présente une fenêtre (23) à travers laquelle est visible le premier élément de mesure (48) du guide (40), et ladite base (20) présente en outre un second élément de mesure (24) placé sur le côté de la fenêtre ;
- le premier élément de mesure (48) et le second élément de mesure (24) comprennent une échelle de graduations réparties le long de l'axe longitudinal (AL) et un repère de référence en correspondance visuelle avec l'échelle de graduations, de manière à mesurer une valeur d'écartement entre le plateau fémoral (31) et le plateau tibial (21).

11. Système chirurgical comprenant un instrument chirurgical (1 ; 100) selon l'une quelconque des revendications 1 à 10, et comprenant en outre un tournevis de serrage (10) apte à coopérer avec la vis de serrage (38) pour assurer son vissage/dévissage, et apte aussi à former un bras de levier, une fois enclenché avec la vis de serrage (38), afin de faire pivoter l'élément fémoral (3) par rapport à l'élément mobile (4) lorsque les mors (34, 35) sont en position desserrée.

12. Système chirurgical comprenant un instrument chirurgical (1 ; 100) selon l'une quelconque des revendications 1 à 10, et comprenant en outre un capteur de force (8) adapté pour être placé de manière amovible sur une face supérieure du plateau fémoral (31), opposée à une face inférieure qui fait face au plateau tibial (21), afin de détecter une ou plusieurs forces exercées par les condyles du fémur sur le plateau fémoral (31).

13. Système chirurgical selon la revendication 12, comprenant en outre un appareil de mesure (9) raccordé au capteur de force (8), par une voie non filaire ou filaire, et prévu pour recevoir les mesures effectuées par le capteur de force (8), dans lequel ledit appareil de mesure (9) est monté de manière amovible sur la base (20) de l'élément tibial statique (2).

14. Système chirurgical selon la revendication 13, dans lequel l'appareil de mesure (9) est muni d'un afficheur (92) ou est raccordé à un afficheur distant, pour un affichage des mesures effectuées par le capteur de force (8).

15. Système chirurgical comprenant un instrument chirurgical (1 ; 100) selon l'une quelconque des revendications 1 à 10, et comprenant en outre un élément de guidage (6) monté de manière amovible sur la base (20) de l'élément tibial statique (2), ledit élément de guidage (6) comprenant plusieurs orifices de guidage (64) pour des perçages dans le fémur.

## Patentansprüche

1. Chirurgisches Instrument (1; 100) für ein Ausbalancieren des Knies im Rahmen einer Vorbereitung für das chirurgische Einsetzen einer Knievollprothese, wobei das chirurgische Instrument (1) umfasst:
- Ein statisches Tibiaelement (2), beinhaltend eine Basis (20), die fest mit einer Tibiaplatte (21) verbunden ist, das dazu vorgesehen ist, auf einem resektierten proximalen Ende der Tibia zum Aufliegen zu kommen;
- ein Femurelement (3), beinhaltend eine Femurplatte (31), das dazu vorgesehen ist, auf den distalen oder hinteren Kondylen des Femurs oder auf einem resektierten distalen Ende des Femurs oder auf einem Ausgleichskeil (7) zum Aufliegen zu kommen;
- ein bewegliches Element (4), beinhaltend eine Führung (40), die translationsbeweglich auf der Basis (20) des statischen Tibiaelements (2) gemäß einer Längsachse (AL) montiert ist, und wobei das Femurelement (3) schwenkend auf dem beweglichen Element (4) gemäß einer anteroposterioren Achse (AP) montiert ist, so dass die Femurplatte (31) sich um die anteroposteriore Achse (AP) in Bezug auf die Tibiaplatte (21), die statisch ist, schwenkt;
- ein Einstellungssystem (5), das zwischen der Führung (40) des beweglichen Elements (4) und der Basis (20) des statischen Tibiaelements (2) gekoppelt ist, für eine Einstellung der Beweglichkeit der Führung (40) in Bezug auf die Basis (20), um für eine Ablenkungsfunktion zu sorgen, indem eine Einstellung des Abstands zwischen der Femurplatte (31) und der Tibiaplatte (21) ermöglicht wird;
wobei das bewegliche Element (4) einen Querarm (43) umfasst, der fest mit der Führung (40) verbunden ist, und das Femurelement (3) einen Femurarm (32) umfasst, der fest mit der Femurplatte (31) verbunden ist;
und wobei das chirurgische Instrument (1; 100) weiterhin ein reversibles Blockiersystem, das zwischen dem Querarm (43) des beweglichen Elements (4) und dem Femurarm (32) des Femurelements (3) gekoppelt ist, zum Immobilisieren des Schwenkens des Femurelements (3) in Bezug auf das bewegliche Element (4) auf reversible Weise umfasst, um den Schwenkwinkel der Femurplatte (31) in Bezug auf die Tibiaplatte (21) auf unterschiedliche Schwenkwinkelwerte festzulegen,
wobei das chirurgische Instrument (1; 100) **dadurch gekennzeichnet ist, dass** das reversible Blockiersystem umfasst:
- eine Klemme (33), die an einem freien Ende des Femurarms (32) vorgesehen ist und die zwei gegenüberliegende Backen (34, 35) umfasst, die zwischen einer geklemmten Position und einer gelösten Position beweglich sind;
- eine Schiene (49), die an einem freien Ende des Querarms (43) des beweglichen Elements (4) vorgesehen ist und die im Inneren der Klemme (33) zwischen den zwei Backen (34, 35) in Eingriff genommen wird;
- ein Klemmelement (38), das mit der Klemme (33) zusammenwirkt, zum Verstellen der Backen (34, 35) zwischen der geklemmten Position und der gelösten Position, wobei das Femurelement (3) in der geklemmten Position in seinem Schwenken in Bezug auf das bewegliche Element (4) immobilisiert ist und wobei in der gelösten Position zugelassen wird, dass das Femurelement (3) sich in Bezug auf das bewegliche Element (4) schwenkt;
und dass das Klemmelement eine Klemmschraube (38) umfasst, die in eine Klemmöffnung (37) geschraubt ist, die durch die zwei Backen (34, 35) verläuft, so dass das Einschrauben der Klemmschraube (38) die Backen (34, 35) zu der geklemmten Position verstellt und das Ausschrauben der Klemmschraube (38) die Backen (34, 35) zu der gelösten Position verstellt;
und dass die Klemmschraube (38) und die Klemmöffnung (37) sich gemäß einer Querachse (AT) erstrecken, die orthogonal zu der anteroposterioren Achse (AP) ist.

2. Chirurgisches Instrument (1; 100) nach Anspruch 1, wobei der Querarm (43) des beweglichen Elements (4) in einem freien Ende endet, das mit einem ersten Kennzeichnungselement (46) versehen ist, und der Femurarm (32) des Femurelements (3) in einem freien Ende endet, das mit einem zweiten Kennzeichnungselement (36) versehen ist, wobei das erste Kennzeichnungselement (46) und das zweite Kennzeichnungselement (36) eine Skala mit Teilstrichen, die um die anteroposteriore Achse (AP) verteilt sind, und eine Referenzmarkierung, die optisch der Skala mit Teilstrichen entspricht, umfassen, um einen Schwenkwinkelwert der Femurplatte (31) in Bezug auf die Tibiaplatte (21) zu messen.

3. Chirurgisches Instrument (1; 100) nach einem der Ansprüche 1 und 2, wobei die Klemme (33) von einem geschlitzten Schieber gebildet wird, in dem ein Schlitz (330) zwischen den zwei Backen (34, 35) vorgesehen ist.

4. Chirurgisches Instrument (1; 100) nach einem der vorhergehenden Ansprüche, wobei die Führung (40) eine Zahnleiste (42) umfasst, die von einer Reihe Zähnen gebildet wird, und das Einstellungssystem (5) ein Zahnrad (50) umfasst, das schwenkend auf der Basis (20) des statischen Tibiaelements (2) montiert ist und das mit der Zahnleiste (42) verzahnt ist.

5. Chirurgisches Instrument (1) nach Anspruch 4, wobei das Einstellungssystem (5) einen reversiblen Verriegelungsmechanismus, der zwischen der Basis (20) des statischen Tibiaelements (2) und dem Zahnrad (50) gekoppelt ist, zum Verriegeln der Drehung des Zahnrads (50) und infolgedessen der Beweglichkeit der Führung (40) in Bezug auf die Basis (20) auf reversible Weise umfasst, um den Abstand zwischen der Femurplatte (31) und der Tibiaplatte (21) festzulegen.

6. Chirurgisches Instrument (1) nach Anspruch 5, wobei der reversible Verriegelungsmechanismus einen Abzug (54) umfasst, der beweglich auf der Basis (20) des statischen Tibiaelements (2) zwischen einer Verriegelungsposition, in der der Abzug (54) mit dem Zahnrad (50) zusammenwirkt, um es in der Drehung zu verriegeln, und einer Entriegelungsposition, in der der Abzug (54) nicht mit dem Zahnrad (50) zusammenwirkt, das sich frei schwenken kann, montiert ist.

7. Chirurgisches Instrument (100) nach Anspruch 4, wobei das Einstellungssystem (5) einen reversiblen Verriegelungsmechanismus, der zwischen der Basis (20) des statischen Tibiaelements (2) und der Zahnleiste (42) gekoppelt ist, zum Verriegeln der Beweglichkeit der Führung (40) in Bezug auf die Basis (20) direkt und auf reversible Weise umfasst, um den Abstand zwischen der Femurplatte (31) und der Tibiaplatte (21) festzulegen.

8. Chirurgisches Instrument (100) nach Anspruch 7, wobei der reversible Verriegelungsmechanismus mindestens einen Riegel (540) umfasst, der schwenkend auf der Basis (20) zwischen einer Verriegelungsposition, in der der Riegel (540) im Eingriff zwischen zwei Zähnen der Zahnleiste (42) steht, um sie in der Drehung zu verriegeln, und einer Entriegelungsposition, in der der Riegel (540) nicht mit den Zähnen der Zahnleiste (42) zusammenwirkt, um zuzulassen, dass sie sich verschiebt, montiert ist.

9. Chirurgisches Instrument (100) nach Anspruch 8, wobei der reversible Verriegelungsmechanismus zwei Riegel (540) umfasst, die schwenkend auf der Basis zwischen einer Verriegelungsposition und einer Entriegelungsposition montiert sind,
und wobei die Riegel (540) gemäß der Längsachse (AL) um einen Abstand, der der Hälfte eines Schritts der Zahnleiste (42) entspricht, voneinander versetzt sind, wobei der Schritt der Zahnleiste (42) dem Abstand zwischen zwei aufeinanderfolgenden Zähnen entspricht, so dass, wenn ein Riegel (540) in der Verriegelungsposition zwischen zwei Zähnen der Zahnleiste (42) ist, der andere Riegel (540) in der Entriegelungsposition ist, indem er im Kontakt mit einem Scheitelpunkt eines Zahns der Zahnleiste (42) steht.

10. Chirurgisches Instrument (1; 100) nach einem der Ansprüche 4 bis 9, wobei:
- die Führung (40) mit einem ersten Messelement (48) versehen ist;
- die Basis (20) des statischen Tibiaelements (2) ein Fenster (23) aufweist, durch das das erste Messelement (48) der Führung (40) sichtbar ist, und die Basis (20) weiterhin ein zweites Messelement (24) aufweist, das an der Seite des Fensters angeordnet ist;
- das erste Messelement (48) und das zweite Messelement (24) eine Skala mit Teilstrichen, die entlang der Längsachse (AL) verteilt sind, und eine Referenzmarkierung, die optisch der Skala mit Teilstrichen entspricht, umfassen, um einen Wert eines Abstands zwischen der Femurplatte (31) und der Tibiaplatte (21) zu messen.

11. Chirurgisches System, umfassend ein chirurgisches Instrument (1; 100) nach einem der Ansprüche 1 bis 10 und weiterhin umfassend einen Klemmschraubendreher (10), der dazu geeignet ist, mit der Klemmschraube (38) zusammenzuwirken, um deren Einschrauben/Ausschrauben sicherzustellen, und außerdem dazu geeignet ist, einen Hebelarm zu bilden, sobald er mit der Klemmschraube (38) eingerastet ist, um das Femurelement (3) in Bezug auf das bewegliche Element (4) schwenken zu lassen, wenn die Backen (34, 35) in der gelösten Position sind.

12. Chirurgisches System, umfassend ein chirurgisches Instrument (1; 100) nach einem der Ansprüche 1 bis 10 und weiterhin umfassend einen Kraftsensor (8), der dazu eingerichtet ist, auf abnehmbare Weise auf einer oberen Fläche der Femurplatte (31), die entgegengesetzt zu einer unteren Fläche ist, die der Tibiaplatte (21) zugewandt ist, angeordnet zu werden, um eine oder mehrere Kräfte zu erkennen, die von den Kondylen des Femurs auf die Femurplatte (31) ausgeübt werden.

13. Chirurgisches System nach Anspruch 12, weiterhin umfassend ein Messgerät (9), das drahtlos oder drahtgebunden mit dem Kraftsensor (8) verbunden ist und dazu vorgesehen ist, Messungen zu empfangen, die von dem Kraftsensor (8) durchgeführt werden, wobei das Messgerät (9) auf abnehmbare Weise auf der Basis (20) des statischen Tibiaelements (2) montiert ist.

14. Chirurgisches System nach Anspruch 13, wobei das Messgerät (9) eine Anzeige (92) umfasst oder mit einer Fernanzeige verbunden ist, um die vom Kraftsensor (8) durchgeführten Messungen anzuzeigen.

15. Chirurgisches System, umfassend ein chirurgisches Instrument (1; 100) nach einem der Ansprüche 1 bis 10 und weiterhin umfassend ein Führungselement (6), das auf abnehmbare Weise auf der Basis (20) des statischen Tibiaelements (2) montiert ist, wobei das Führungselement (6) mehrere Führungsöffnungen (64) für Bohrungen in das Femur umfasst.

## Claims

1. A surgical instrument (1; 100) for knee balancing in the context of a preparation of surgical setting of a total knee prosthesis, said surgical instrument (1) comprising:
- a static tibial element (2) including a base (20) secured to a tibial paddle (21) intended to bear on a resected proximal end of the tibia;
- a femoral element (3) including a femoral paddle (31) intended to bear on the distal or posterior condyles of the femur or on a resected distal end of the femur or on a compensation wedge (7);
- a movable element (4) including a guide (40) mounted movable in translation on the base (20) of the static tibial element (2) according to a longitudinal axis (AL), and wherein the femoral element (3) is pivotally mounted on said movable element (4) according to an anterior-posterior axis (AP), so that the femoral paddle (31) pivots about said anterior-posterior axis (AP) relative to the tibial paddle (21) which is static;
- a setting system (5) coupled between the guide (40) of the movable element (4) and the base (20) of the static tibial element (2) for setting the mobility of said guide (40) relative to said base (20) in order to provide a distraction function while enabling a setting of the spacing between the femoral paddle (31) and the tibial paddle (21);
wherein the movable element (4) comprises a transverse arm (43) secured to the guide (40), and the femoral element (3) comprises a femoral arm (32) secured to the femoral paddle (31);
and wherein the surgical instrument (1; 100) further comprises a reversible blocking system coupled between the transverse arm (43) of the movable element (4) and the femoral arm (32) of the femoral element (3) to reversibly immobilize the pivoting of the femoral element (3) with respect to the movable element (4) in order to set the pivot angle of the femoral paddle (31) with respect to the tibial paddle (21) at different pivot angle values;
said surgical instrument (1; 100) being **characterized in that** the reversible blocking system comprises:
- a jaw (33) which is provided on a free end of the femoral arm (32), and which comprises two clamps (34, 35) opposite each other and movable between a clamped position and an unclamped position;
- a rail (49) which is provided on a free end of the transverse arm (43) of the movable element (4) and which is engaged into the jaw (33) between the two clamps (34, 35);
- a clamping element (38) cooperating with the jaw (33) so as to displace the clamps (34, 35) between the clamped position and the unclamped position, such that, when in the clamped position, the femoral element (3) is pivotally immobilized with respect to the movable element (4), and, when in the unclamped position, the femoral element (3) is enabled to pivot relative to the movable element (4);
**in that** said clamping element comprises a clamping screw (38) screwed into a clamping orifice (37) which passes through the two clamps (34, 35), so that screwing of the clamping screw (38) displaces the clamps (34, 35) toward the clamped position and unscrewing of the clamping screw (38) displaces the clamps (34, 35) toward the unclamped position;
and **in that** the clamping screw (38) and the clamping orifice (37) extend according to a transverse axis (AT) orthogonal to the anterior-posterior axis (AP).

2. The surgical instrument (1; 100) according to claim 1, wherein the transverse arm (43) of the movable element (4) terminates in a free end provided with a first reference element (46), and the femoral arm (32) of the femoral element (3) terminates in a free end provided with a second reference element (36), wherein the first reference element (46) and the second reference element (36) comprise a graduated scale distributed around the anterior-posterior axis (AP) and a reference marking visually matching with the graduated scale, so as to measure a value of the pivot angle of the femoral paddle (31) with respect to the tibial paddle (21).

3. The surgical instrument (1; 100) according to any one of the claims 1 and 2, wherein the jaw (33) is formed by a slotted slide in which a slot (330) is provided between the two clamps (34, 35).

4. The surgical instrument (1; 100) according to any one of the preceding claims, wherein the guide (40) comprises a rack (42) formed by a series of teeth, and the setting system (5) comprises a pinion (50) which is pivotally mounted on the base (20) of the static tibial element (2) and which meshes with the rack (42).

5. The surgical instrument (1) according to claim 4, wherein the setting system (5) comprises a reversible locking mechanism coupled between the base (20) of the static tibial element (2) and the pinion (50) so as to reversibly lock the rotation of the pinion (50) and consequently the mobility of the guide (40) relative to the base (20), in order to set the spacing between the femoral paddle (31) and the tibial paddle (21).

6. The surgical instrument (1) according to claim 5, wherein the reversible locking mechanism comprises a latch (54) movably mounted on the base (20) of the static tibial element (2) between a locking position in which the latch (54) cooperates with the pinion (50) so as to rotatably lock it, and an unlocking position in which the latch (54) does not cooperate with the pinion (50) which is free to pivot.

7. The surgical instrument (100) according to claim 4, wherein the setting system (5) comprises a reversible locking mechanism coupled between the base (20) of the static tibial element (2) and the rack (42) so as to reversibly and directly lock the mobility of the guide (40) relative to the base (20), in order to set the spacing between the femoral paddle (31) and the tibial paddle (21).

8. The surgical instrument (100) according to claim 7, wherein the reversible locking mechanism comprises at least one bolt (540) pivotally mounted on the base (20) between a locking position in which the bolt (540) meshes between two teeth of the rack (42) to lock it in translation, and an unlocking position in which the bolt (540) does not mesh with the teeth of the rack (42) to let it translate.

9. The surgical instrument (100) according to claim 8, wherein the reversible locking mechanism comprises two bolts (540) pivotally mounted on the base (20) between a locking position and an unlocking position,
and wherein said bolts (540) are offset from each other along the longitudinal axis (AL) by a difference which corresponding to half a pitch of the rack (42), the pitch of the rack (42) corresponding to the gap between two successive teeth, such as when a bolt (540) is in the locking position between two teeth of the rack (42) then the other bolt (540) is in the unlocking position by being in contact with a top of one teeth of the rack (42).

10. The surgical instrument (1; 100) according to any one of claims 4 to 9, wherein:
- the guide (40) is provided with a first measuring element (48);
- the base (20) of the static tibial element (2) presents an aperture (23) through which the first measuring element (48) of the guide (40) is visible, and said base (20) further presents a second measuring element (24) placed at the aperture side;
- the first measuring element (48) and the second measuring element (24) comprise a graduated scale distributed along the longitudinal axis (AL) and a reference marking visually matching with the graduated scale, so as to measure a value of the spacing between the femoral paddle (31) and the tibial paddle (21).

11. A surgical system comprising a surgical instrument (1; 100) according to any one of claims 1 to 10, and further comprising a clamping screwdriver (10) capable of cooperating with the clamping screw (38) so as to ensure the screwing/unscrewing thereof, and also capable of forming a lever arm, once engaged with the clamping screw (38), in order to make the femoral element (3) pivot relative to the movable element (4) when the clamps (34, 35) are in the unclamped position.

12. A surgical system comprising a surgical instrument (1; 100) according to any one of claims 1 to 10, and further comprising a force sensor (8) adapted to be removably placed on an upper face of the femoral paddle (31), opposite to a lower face which faces the tibial paddle (21), in order to detect one or several force(s) exerted by the condyles of the femur on the femoral paddle (31).

13. The surgical system according to claim 12, further comprising a measuring apparatus (9) connected to the force sensor (8), via a wireless or wired link, and intended to receive the measurements performed by the force sensor (8), wherein said measuring apparatus (9) is removably mounted on the base (20) of the static tibial element (2).

14. The surgical system according to claim 13, wherein the measuring apparatus (9) is provided with a display (92) or is connected to a distant display, to display the measurements performed by the force sensor (8).

15. A surgical system comprising a surgical instrument (1; 100) according to any one of claims 1 to 10, and further comprising a guide element (6) removably mounted on the base (20) of the static tibial element (2), said guide element (6) comprising several guide orifices (64) for drills in the femur.
